(19) Europäisches Patentamt
European Patent Office
Office européen des brevets

(11) **EP 4 343 323 A1**

(12) **EUROPEAN PATENT APPLICATION**

(43) Date of publication:
**27.03.2024 Bulletin 2024/13**

(21) Application number: **23174366.7**

(22) Date of filing: **19.05.2023**

(51) International Patent Classification (IPC):
**G01N 30/88** (2006.01)   **G16H 50/20** (2018.01)
**G01N 30/86** (2006.01)

(52) Cooperative Patent Classification (CPC):
**G01N 30/88; G16H 50/20;** G01N 30/8679;
G01N 2030/8822

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **23.05.2022   JP 2022084016
11.05.2023   JP 2023078766**

(71) Applicant: **ARKRAY, Inc.**
**Kyoto-shi, Kyoto 601-8045 (JP)**

(72) Inventor: **NAKAYAMA, Yusuke**
**Kyoto, 602-0008 (JP)**

(74) Representative: **Dehns**
**St. Bride's House**
**10 Salisbury Square**
**London EC4Y 8JD (GB)**

(54) **HEMOGLOBIN A1C MEASUREMENT METHOD, HEMOGLOBIN ANALYZER, AND NON-TRANSITORY COMPUTER-READABLE RECORDING MEDIUM**

(57)   There is provided a stable glycohemoglobin measurement method. In the method, a processor (100A) executes a process to separate hemoglobin in blood to acquire a hemoglobin A1c peak derived from stable glycohemoglobin, hemoglobin A peaks including fractions derived from hemoglobin A, and hemoglobin-derived peaks fractionated other than the hemoglobin A peaks and find the value of the stable glycohemoglobin by performing a correction calculation to lower, based on the area of a peak selected from among the hemoglobin-derived peaks fractionated other than the hemoglobin A peaks, a value obtained by dividing the area of the hemoglobin A1c peak by the areas of the hemoglobin A peaks.

FIG.2

EP 4 343 323 A1

**Description**

BACKGROUND

Technical Field

The present invention relates to a stable glycohemoglobin measurement method, a stable glycohemoglobin measurement apparatus, and a non-transitory computer-readable recording medium.

Related Art

Technologies relating to methods of analyzing hemoglobin in blood using high-performance liquid chromatography (HPLC) have been disclosed. When analyzing hemoglobin in blood, hemoglobin A1c (HbA1c) in particular is, together with the blood glucose level (the concentration of glucose in the blood), an important marker in testing for diabetes, so it has become able to be measured by hemoglobin analyzers.

Japanese Patent Application Laid-open (JP-A) No. 2017-198666 discloses that an HPLC hemoglobin analyzer can be used to fractionate hemoglobin components such as HbA1c to acquire the peak of each component. The record of the components fractionated by HPLC is called a chromatogram, and JP-ANo. 2017-198666 discloses that HbS and HbC, which are variant hemoglobins, can be separated in addition to HbA0 and HbA1c. Furthermore, JP-ANo. 2001-228133 discloses a technology that calculates stable HbA1c. In JP-ANo. 2017-198666, it is stated that in the case of a blood specimen including HbS and HbC, if a separation condition for separating the HbA0 peak and the other HbS and HbC peaks is set, when calculating the percentage (%) of HbA1c a more accurate stable HbA1c can be calculated by removing variant hemoglobin components other than HbA from the HbA0 peak.

JP-A No. 2009-109231 discloses a method of analyzing hemoglobin in blood using not HPLC but capillary electrophoresis using electrokinetic chromatography based on the same principle of cation exchange. The record of the components fractionated by electrophoresis is called a pherogram, and in technologies that fractionate hemoglobin in blood, a pherogram can be handled in the same way as an HPLC chromatogram. In JP-A No. 2009-109231, it is stated that, to calculate the value of the percentage (%) of each Hb component such as HbA1c from the obtained electropherogram, a calculation method is used where the peak area of each Hb component is divided by a peak area b obtained by excluding the peak area of HbA2 and the peak area of HbF from the peak area of all the Hb components.

JP-A No. 2014-235023 discloses a method of quantifying HbF using HPLC. In JP-A No. 2014-235023, the clinical significance of HbF and HbA2 and their prevalence in hemoglobin are described. In particular, it is shown that the HbA2 value in healthy subjects is very small, ranging from 2% to 3.5%, and in thalassemia patients, it needs to be quantified in the concentration range of 2% to 7%.

However, in Japan, China, and other East Asian countries, the incidence rate of thalassemia is low, so there is also less of a demand to quantify HbA2 and the effect on HbA1c values in healthy subjects is also small. For that reason, giving priority to processing speed in measuring HbA1c, HPLC hemoglobin analyzers for HbA1c value measurement predominantly output, without separating, HbA2 so that it overlaps the HbA0 peak.

Among HPLC hemoglobin analyzers for HbA1c value measurement in Japan, China, and other East Asian countries, those that do not fractionate HbA2 but output HbA0 and HbA2 as overlapping peaks are predominant. Reasons for this are, as mentioned above, that the incidence rate of thalassemia is low and HbA2 values are low in healthy subjects, ranging from 2% to 3.5%, and furthermore that both individual differences and fluctuations are small and, in a chromatogram, the extent of the percentage of the area of the HbA2 peak is low relative to the area of the peak of HbA0 itself, so no matter the specimen the HbA2 value has been regarded as something included in the total area derived from hemoglobin A on a chromatogram and has come to be used by outputting the HbA1c value.

Specifically, the aforementioned HPLC hemoglobin analyzers for HbA1c value measurement that are predominant in Japan and other East Asian countries use an equation such as, for example, equation (A). That is, the HbA1c value (A1c %) is found by dividing a numerator consisting of the area of the HbA1c peak that can be fractionated on a chromatogram and represented by "A1c" in the calculation by a denominator consisting of the measurement value of the area of the HbF peak that can be fractionated on a chromatogram and represented by "F" subtracted from the sum total of the areas of the fractionated peaks derived from the hemoglobins that can be fractionated on a chromatogram and represented by "All Hb" (the total area, i.e., an integral value obtained by integrating the chromatogram). The measurement value of all the hemoglobins is an area (integral value) corresponding to all hemoglobin-derived components in the chromatogram, and includes also the HbA2 component. The measurement value of HbF is an area (integral value) corresponding to HbF in the chromatogram.

$$A1c\% = \frac{A1c}{\text{All Hb} - F} \times 100 \quad \cdots \quad (A)$$

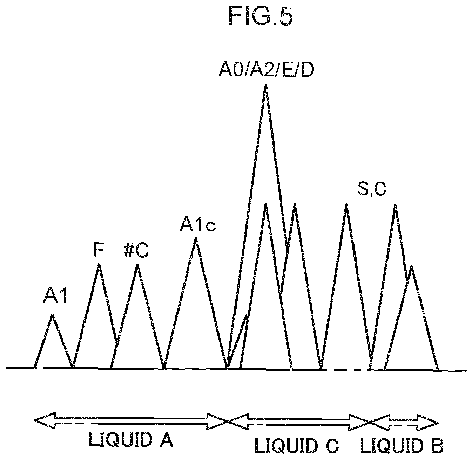

Furthermore, as another example of the aforementioned HPLC hemoglobin analyzers for HbA1c value measurement that are predominant in Japan and other East Asian countries, analyzers that can separate variant hemoglobins but cannot separate the HbA2 component are also in common use. FIG. 5 is an example of a chromatogram in which the HbA2 component cannot be separated, and as shown in FIG. 5, the HbA0 peak and the HbA2 peak have overlapped in the chromatogram. This kind of hemoglobin analyzer specifically has found the value of stable glycohemoglobin (A1c%) based on equation (B) below. In equation (B), "A1c" is the area of the HbA1c peak that can be fractionated on the chromatogram, and "All Hb" is the sum total of the areas of fractionated peaks derived from the hemoglobins that can be fractionated on the chromatogram (the total area, i.e., an integral value obtained by integrating the chromatogram). "F" is the area of the HbF peak that can be fractionated on the chromatogram. "Other Hb" is the sum total of the areas of the peaks of variant hemoglobins such as HbS and HbC that can be fractionated on the chromatogram. That is, analyzers that can separate variant hemoglobins but cannot separate the HbA2 component have divided a numerator consisting of the area of the HbA1c peak in the calculation by a denominator consisting of an area including the HbA2 component.

$$A1c\% = \frac{A1c}{(\text{All Hb}) - (F + \text{ Other Hb})} \times 100 \quad \cdots \quad (B)$$

In this way, no matter the specimen, the area of the HbA2 peak has been included in the "All Hb" term in equation (A) and equation (B), and the HbA2 value has come to be used by outputting the HbA1c value.

Meanwhile, owing to remarkably greater human mobility worldwide, in Japan or China or other East Asian countries also, opportunities for non-native persons to consult doctors are increasing at a rapid rate. Moreover, because of eluent types or feed methods and camera innovations, even among HPLC hemoglobin analyzers for HbA1c value measurement, analyzers that can fractionate HbA2 in a short amount of time have been devised (see JP-A No. 2021-056174).

In accordance with this, the methods of JP-A No. 2001-228133 and JP-A No. 2009-109231 are now able to fractionate HbA2 and variant hemoglobin components, so against the backdrop that clinically the HbA1c value is the percentage of the HbA1c component in the HbA components, an equation such as, for example, equation (C) can be used. In equation (C), "A1c" is the area of the HbA1c peak that can be fractionated on the chromatogram, "All Hb" is the sum total of the areas of the fractionated peaks derived from the hemoglobins that can be fractionated on the chromatogram (the total area, i.e., an integral value obtained by integrating the chromatogram), "A2" is the area of the A2 peak that can be fractionated on the chromatogram, "F" is the area of the HbF peak that can be fractionated on the chromatogram, and "Other Hb" is the sum total of the areas of the peaks of variant hemoglobins such as HbS and HbC that can be fractionated on the chromatogram. That is, because the HbA components do not include the HbF component, the HbA2 component, and variant hemoglobin components, in the denominator the difference of "F," "A2," and "Other Hb" has been taken from "All Hb." That is, in equation (C), which is an arithmetic equation for obtaining the HbA1c value, the area of the HbA1c peak, which is the numerator, is divided by a value not including the area of the HbA2 peak in the denominator.

$$A1c\% = \frac{A1c}{(\text{All Hb}) - (F + A2 + \text{ Other Hb})} \times 100 \quad \cdots \quad (C)$$

However, in the HPLC hemoglobin analyzers for HbA1c value measurement that are predominant in Japan and other East Asian countries, the HbA1c value has been calculated using equation (A) and equation (B), so in the case of calculating the HbA1c value using equation (C), in which the denominator is a value not including the HbA2 component, the HbA1c value becomes higher than it does in the predominant case where the denominator is a value including the HbA2 component. For that reason, in a case where the analyzer is replaced, if there is a difference in the calculation method between the new and old analyzers, the HbA1c value becomes higher, so more patients are determined to be hyperglycemic or suspected of being hyperglycemic, causing confusion in users at hospitals and elsewhere.

SUMMARY

The present invention has been made in view of the above considerations, and at least preferred embodiments provide a stable glycohemoglobin measurement method, a stable glycohemoglobin measurement apparatus, and a non-transitory computer-readable recording medium that avoid higher values when measuring stable glycohemoglobin values using a hemoglobin component that is now able to be fractionated.

An aspect of this invention provides a stable glycohemoglobin measurement method whereby a processor executes a process to separate hemoglobin in blood to acquire a hemoglobin A1c peak derived from stable glycohemoglobin, hemoglobin A peaks including fractions derived from hemoglobin A, and hemoglobin-derived peaks fractionated other than the hemoglobin A peaks and find the value of the stable glycohemoglobin by performing a correction calculation to lower (reduce), based on the area of a peak selected from among the hemoglobin-derived peaks fractionated other than the hemoglobin A peaks, a value obtained by dividing the area of the hemoglobin A1c peak by the areas of the hemoglobin A peaks.

According to the present invention, there can be provided a stable glycohemoglobin measurement method, a stable glycohemoglobin measurement apparatus, and a non-transitory computer-readable recording medium that avoid higher values when measuring stable glycohemoglobin values using fractionated hemoglobin components.

BRIEF DESCRIPTION OF THE DRAWINGS

An exemplary embodiment of the present invention will be described in detail based on the following figures, wherein:

FIG. 1 is a diagram showing the schematic configuration of an HPLC apparatus utilizing high-performance liquid chromatography;
FIG. 2 is a block diagram showing an example of hardware configurations of a control system of the HPLC apparatus;
FIG. 3 is a block diagram showing an example of functional configurations of a control unit;
FIG. 4 is a drawing showing an example of a chromatogram obtained by the HPLC apparatus;
FIG. 5 is an example of a chromatogram in which the HbA2 component is unable to be separated; and
FIG. 6 is a flowchart showing the flow of a stable glycohemoglobin measurement process executed by the control unit.

DETAILED DESCRIPTION

An example of an embodiment of the present invention will be described below with reference to the drawings. It will be noted that identical or equivalent constituent elements and parts are assigned identical reference signs in the drawings. Furthermore, dimensional ratios in the drawings are exaggerated for the sake of description and may differ from actual ratios. Furthermore, herein, hemoglobin is sometimes abbreviated as "Hb." Consequently, hemoglobin A1c is the same as HbA1c.

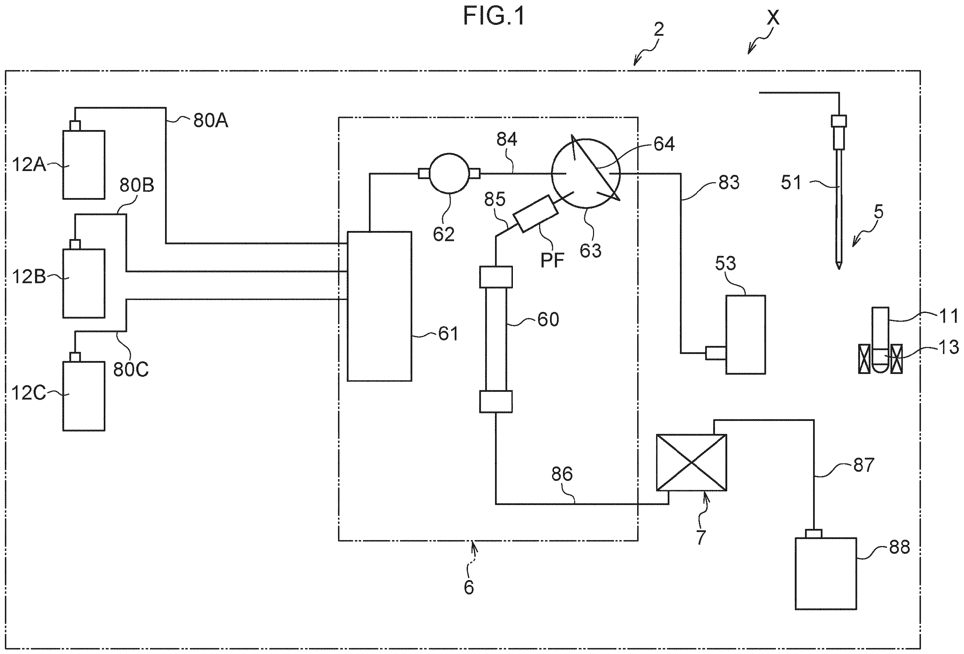

FIG. 1 is a diagram showing the schematic configuration of an HPLC apparatus X utilizing high-performance liquid chromatography (HPLC).

The HPLC apparatus X is configured to set a blood collection tube 11 and automatically measure the concentration of glycohemoglobin (HbA1c) in whole blood. The HPLC apparatus X has an apparatus body 2 including plural eluent bottles 12A, 12B, 12C (in FIG. 1, three eluent bottles).

The eluent bottles 12A to 12C hold eluents A to C that are to be supplied to an analytical column 60 described later. The eluents have, for example, different compositions, component ratios, pHs, and osmotic pressures in accordance with their use.

The apparatus body 2 has a sample preparation unit 5, an analyzer unit 6, and a photometer unit 7.

The blood collection tube 11 is, for example, stored in a rack (not shown in the drawings), and is configured to be moved to a position at which blood in the blood collection tube 11 can be collected by a nozzle 51 of the sample preparation unit 5 described later.

The sample preparation unit 5 is for preparing, from the blood collected from the blood collection tube 11, a sample to be introduced to the analytical column 60. The sample preparation unit 5 has the nozzle 51 and a dilution tank 53.

The nozzle 51 is for collecting various types of liquids including a blood sample 13 in the blood collection tube 11, and can suck and discharge liquids and can move in the up and down direction and the horizontal direction. The operation of the nozzle 51 is controlled by a control unit 100 described later.

The analyzer unit 6 is for controlling the adsorption and desorption of biocomponents with respect to a filler in the analytical column 60 and supplying various types of biocomponents to the photometer unit 7. The set temperature in the analyzer unit 6 is, for example, about 40 °C. The analytical column 60 holds the filler for selectively adsorbing hemoglobins in the sample. As the filler, for example, a copolymer of methacrylic acid and methacrylic acid ester is used.

The analyzer unit 6 has, in addition to the analytical column 60, a manifold 61, a feed pump 62, and an injection valve 63.

The manifold 61 is for selectively supplying an eluent from a specific eluent bottle of the plural eluent bottles 12A to 12C to the analytical column 60. The manifold 61 is connected via tubes 80A to 80C to the eluent bottles 12A, 12B, 12C and is connected via a tube 84 to the injection valve 63.

The feed pump 62 is for applying motive force for moving the eluent to the injection valve 63 and is provided in the middle of the tube 84.

The HPLC apparatus X measures HbA1c and variant hemoglobins in the blood sample 13 by sequentially feeding in a predetermined order the plural types of eluents to the analytical column 60. The plural types of eluents are, in the present embodiment, liquid A to liquid C. Liquid A is prepared in the eluent bottle 12A, and is an eluent for eluting HbA1c and for equilibrating the analytical column 60. Liquid B is prepared in the eluent bottle 12B, and is an eluent for eluting all the hemoglobin remaining in the analytical column 60, that is, an eluent for washing the analytical column 60. Liquid C is prepared in the eluent bottle 12C, and is an eluent for eluting hemoglobins other than HbA1c after HbA1c has been eluted. It will be noted that in descending order of hemoglobin eluting power, the liquids rank in the order of liquid B, liquid C, and liquid A.

The injection valve 63 can collect a fixed amount of the sample and introduce the sample to the analytical column 60, and has plural inlet ports and outlet ports (not shown in the drawings). The injection valve 63 is connected to the dilution tank 53 by a tube 83. Connected to the injection valve 63 is an injection loop 64. The injection loop 64 can hold a certain amount of liquid (for example, several $\mu$L), and by appropriately switching the injection valve 63, it can select a state in which the injection loop 64 communicates with the dilution tank 53 so that the sample is supplied from the dilution tank 53 to the injection loop 64 and a state in which the injection loop 64 communicates with the analytical column 60 via a prefilter PF and a tube 85 so that the sample is introduced from the injection loop 64 to the analytical column 60. As the injection valve 63, for example, a six-port valve can be used. It will be noted that the prefilter PF is a filter for filtering the sample and the eluents.

The photometer unit 7 is for optically detecting hemoglobins included in the eluents supplied thereto via a tube 86 from the analytical column 60, and is connected via a tube 87 to a waste liquid tank 88 for discharging liquid desorbed from the analytical column 60. In the photometer unit 7, the desorbed liquid is continuously irradiated with light, and the light reception results (absorbances) are output to the control unit 100. Then, in the control unit 100, a chromatogram is calculated based on the light reception results.

A detailed method of separating hemoglobin using the HPLC apparatus X is disclosed in, for example, JP-ANo. 2017-198666, so detailed description thereof will be omitted here, but the hemoglobin can be separated by supplying, in a predetermined order and time, liquid A to liquid C to the analytical column 60.

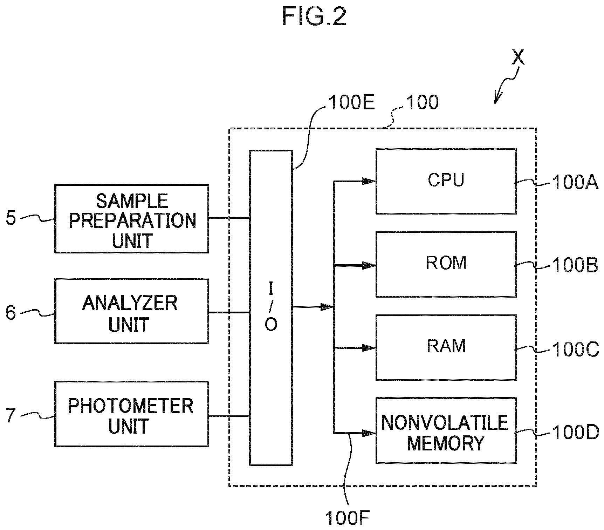

FIG. 2 is a block diagram showing an example of hardware configurations of a control system of the HPLC apparatus X.

As shown in FIG. 2, the HPLC apparatus X includes a control unit 100. The control unit 100 has a central processing unit (CPU) 100A, a read-only memory (ROM) 100B, a random-access memory (RAM) 100C, a nonvolatile memory 100D, and an input/output interface (I/O) 100E that are connected to each other via a bus 100F. The HPLC apparatus X also includes an operation unit (not shown in the drawings) that receives input from an operator.

The CPU 100Ais a central processing unit, executes various types of programs, and controls each part of the HPLC apparatus X. That is, the CPU 100A reads programs from the ROM 100B or the nonvolatile memory 100D and executes the programs using the RAM 100C as a workspace. The CPU 100A controls each configuration and performs various types of arithmetic processing in accordance with the programs recorded in the ROM 100B or the nonvolatile memory 100D. In the present embodiment, the ROM 100B or the nonvolatile memory 100D stores a stable glycohemoglobin measurement program for measuring the amount of stable glycohemoglobin.

The ROM 100B stores various types of programs and various types of data. The RAM 100C temporarily stores programs or data as a workspace. The nonvolatile memory 100D stores various types of programs, including an operating system, and various types of data.

To the input/output interface 100E are connected the sample preparation unit 5, the analyzer unit 6, and the photometer unit 7.

When executing the stable glycohemoglobin measurement program, the control unit 100 uses the above hardware resources to realize various types of functions. Next, functional configurations realized by the control unit 100 will be described.

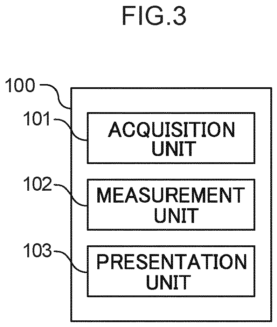

FIG. 3 is a block diagram showing an example of the functional configurations of the control unit 100.

As shown in FIG. 3, the control unit 100 has, as functional configurations, an acquisition unit 101, a measurement unit 102, and a presentation unit 103. Each of these functional configurations is realized by the CPU 100A reading and executing the stable glycohemoglobin measurement program stored in the ROM 100B or the nonvolatile memory 100D.

The acquisition unit 101 controls the sample preparation unit 5, the analyzer unit 6, and the photometer unit 7 to implement chromatography. Then, the acquisition unit 101 acquires absorbances, which are optical measurement values output by the photometer unit 7, and creates a chromatogram using the acquired absorbances and the elapsed time since the start of measurement. Then, the acquisition unit 101 acquires the peak of each component of the hemoglobin from the chromatogram based on the light reception results acquired by the photometer unit 7.

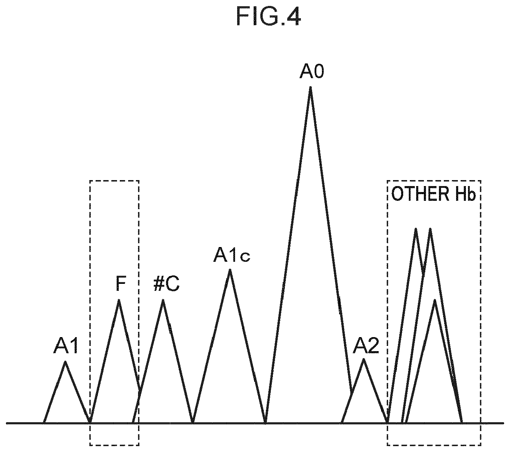

FIG. 4 is a drawing showing an example of a chromatogram obtained by the HPLC apparatus X pertaining to the present embodiment. The chromatogram, as shown in FIG. 4, is a graph representing the relationship between the elapsed time since the start of measurement and the absorbances serving as the light reception results. Which hemoglobins have been detected can be known by the positions in which the peaks in the chromatogram appear, and the concentrations of the hemoglobins can be known by the integral values of the absorbances in the peak portions, that is, the sizes of the areas of the peak portions.

The HPLC apparatus X pertaining to the present embodiment, as shown in FIG. 4, can obtain a chromatogram in which peaks of the HbA1 component, the HbF component, the Hb#C (unstable HbA1c) component, the HbA1c (stable HbA1c) component, the HbA0 component, the HbA2 component, and other Hb components appear. The acquisition unit 101 typically acquires, from the results of separating the hemoglobin in the blood included in the eluate from the analytical column 60, that is, the chromatogram, the HbA1 peak, the Hb#C peak, the HbA1c peak, and the HbA0 peak as an HbA1c peak derived from stable glycohemoglobin and HbA peaks including fractions derived from HbA. The acquisition unit 101 further acquires the HbF peak and the HbA2 peak as Hb-derived peaks fractionated other than hemoglobin A. The HbA1c peak corresponds to the hemoglobin A1c peak. The HbA peaks correspond to the hemoglobin A peaks . The HbF peak and the HbA2 peak correspond to hemoglobin-derived peaks fractionated other than the hemoglobin A peaks. In other words, the HPLC apparatus X is an apparatus capable of separating hemoglobin derived from HbA0, HbF, HbA2, and other Hb included in the blood sample.

The acquisition unit 101 may further acquire Hb peaks corresponding to peaks of variant hemoglobins (the HbS component, the HbC component, etc.) in the blood included in the eluate from the analytical column 60. The Hb peaks corresponding to the peaks of the variant hemoglobins correspond to variant hemoglobin peaks.

The measurement unit 102 calculates, based on the chromatogram acquired by the acquisition unit 101, the areas of the respective peaks including the area of the HbA1c peak, the areas of the HbA peaks, and the area of a peak selected from among the hemoglobin-derived peaks fractionated other than the HbA peaks. A known method can be employed for the calculation method. As an example, in the case of the area of the HbA1c peak, the area of the HbA1c peak can be calculated by calculating the area of the region corresponding to the HbA1c peak in the chromatogram acquired by the acquisition unit 101, that is, an integral value obtained by integrating that region of the chromatogram. Furthermore, in a case where different peaks overlap, the measurement unit 102 estimates the regions corresponding to the respective peaks in the chromatogram acquired by the acquisition unit 101 and uses a predetermined estimation calculation method to calculate the areas of the respective peaks. A known method can be employed for the estimation calculation method. Then, the measurement unit 102 finds the value of the stable glycohemoglobin (the HbA1c value) by dividing the area of the HbA1c peak by a value obtained by adding together the areas of the HbA peaks and the area of the peak selected from among the hemoglobin-derived peaks fractionated other than the HbA peaks. Here, the peak selected from among the hemoglobin-derived peaks may be the HbA2 peak. Furthermore, the areas may be relative values or absolute values. The relative values may be ratios relative to the whole of the area of the chromatogram, ratios relative to the whole of the peak areas relating to the hemoglobins occupying the chromatogram, or ratios relative to the area of a specific peak (e.g., the HbA0 peak), or may be values corresponding to the heights of the peaks.

The measurement unit 102 specifically finds the value of the stable glycohemoglobin (the HbA1c value) represented by A1c% based on equation (1) below. Equation (1) is an example of a first arithmetic equation. In equation (1), "A1c" is the area of the HbA1c peak that can be fractionated on the chromatogram, "HbA" is the sum total of the areas of the hemoglobin A peaks including fractions derived from hemoglobin A that can be fractionated on the chromatogram (i.e., not including the areas of the fractionated peaks of HbF, HbA2, and variant hemoglobins), and "A2" is the area of the HbA2 peak that can be fractionated on the chromatogram. In other words, "HbA" is the sum total of the peak area of the HbA1 fraction, the peak area of the Hb#C fraction, the peak area of the HbA1c fraction, the peak areas of modified Hb fractions derived from HbA, and the peak area of the HbA0 fraction.

$$A1c\% = \frac{A1c}{HbA + A2} \times 100 \quad \cdot \quad \cdot \quad \cdot \quad (1)$$

Equation (1) above can also be expressed as equation (2) below, and the measurement unit 102 may find the value of the stable glycohemoglobin (the HbA1c value) represented by "A1c%" based on equation (2). Equation (2) is also an example of the first arithmetic equation. In equation (2), "A1c" is the area of the HbA1c peak that can be fractionated on the chromatogram, "All Hb" is the sum total of the areas of the fractionated peaks derived from the hemoglobins that can be fractionated on the chromatogram (the total area, i.e., an integral value obtained by integrating the chromatogram), "F" is the area of the HbF peak that can be fractionated on the chromatogram, and "Other Hb" is the sum total of the peaks of the variant hemoglobins such as HbS and HbC that can be fractionated on the chromatogram. Here, the sum total of the areas of the hemoglobin A peaks including fractions derived from hemoglobin A corresponds to a value obtained by subtracting "A2" of equation (1), which is the area of the HbA2 peak that can be fractionated on the chromatogram, from the denominator "(All Hb) - (F + Other Hb)" of equation (2).

$$A1c\% = \frac{A1c}{(\text{All Hb}) - (F + \text{Other Hb})} \times 100 \quad \cdot \cdot \cdot \quad (2)$$

The presentation unit 103 presents the HbA1c value that the measurement unit 102 has calculated using equation (1) or equation (2). When presenting the HbA1c value, the presentation unit 103 may, if the HbA1c value exceeds a predetermined threshold, also present an indication that the patient is hyperglycemic or suspected of being hyperglycemic. It will be noted that the HPLC apparatus X may include a display unit such as a display for the presentation unit 103 to present the HbA1c value (not shown in the drawings).

Here, as mentioned above, HPLC hemoglobin analyzers for HbA1c value measurement that cannot separate the HbA2 component and variant hemoglobins have found the HbA1c value as in equation (A) above. HPLC hemoglobin analyzers for HbA1c value measurement that can separate some variant hemoglobins but cannot separate the HbA2 component have found the HbA1c value as in equation (B) above.

It will be noted that although equation (B) and equation (2) are the same, the components from which the value of "All Hb" is derived are different in that "All Hb" in equation (2) is a value found by performing a calculation to add the area of the fractionated HbA2 peak because the HbA2 peak can be fractionated on the chromatogram, whereas "All Hb" in equation (B) is a value found by performing a calculation using as is the HbA0 peak regarded as including the HbA2 peak because the HbA2 peak could not be fractionated on the chromatogram.

Here, as mentioned above, when the HbA2 component and variant hemoglobin components become able to be fractionated, the HbA1c value can be calculated based on equation (3) below as in the methods disclosed in JP-ANo. 2001-228133 and JP-ANo. 2009-109231, for example. It will be noted that equation (3) is the same arithmetic equation as equation (C) above, and the components from which the values of each of the terms are derived are also the same.

$$A1c\% = \frac{A1c}{(\text{All Hb}) - (F + A2 + \text{Other Hb})} \times 100 \quad \cdot \cdot \cdot \quad (3)$$

In conventional HPLC hemoglobin analyzers for HbA1c value measurement that are predominant in Japan and other East Asian countries, as in equation (B) the "All Hb" term in the denominator includes the area of the peak corresponding to the HbA2 component, and in the calculation, the HbA1c value has been found by dividing "A1c", which is the HbA1c component in the numerator, by a value including the HbA2 component in the denominator. By contrast, in the case of hemoglobin analyzers for HbA1c value measurement that can find the HbA1c value using equation (3), the "All Hb" term includes the area of the peak corresponding to the HbA2 component, and because it is now possible to fractionate the HbA2 component on the chromatogram, the difference of the peak area corresponding to the HbA2 component can be taken from the "All Hb" term. That is, equation (3) is an equation where, in the calculation for obtaining the HbA1c value, "A1c", which is the HbA1c component in the numerator, is divided by a value not including the HbA2 component in the denominator. Clinically, the HbA1c value found using equation (3) is more accurate than the HbA1c value found using equation (B).

Comparing equation (B) and equation (3) in this way, equation (B) includes the area of the peak corresponding to the HbA2 component in the denominator, while equation (3) does not include the area of the peak corresponding to the HbA2 component in the denominator. That is, comparing the HbA1c values calculated based on equation (B) and equation (3) using the areas of the peaks based on the fractions in the same chromatogram, the HbA1c value calculated using equation (3) comes out higher (becomes a higher value). For that reason, in a case where an apparatus that finds the HbA1c value using equation (B) is replaced with an apparatus that finds the HbA1c value using equation (3), the HbA1c value becomes higher, so more patients are determined to be hyperglycemic or suspected of being hyperglycemic, causing confusion in users at hospitals and elsewhere.

Thus, the measurement unit 102 pertaining to the present embodiment calculates the HbA1c value using equation (1) or equation (2). That is, the HbA1c value calculated by the measurement unit 102 is found using an equation where the denominator includes the area of the HbA2 peak that can be fractionated on the chromatogram, which is the area in the chromatogram based on the concentration of HbA2 in the specimen. For that reason, the HPLC apparatus X pertaining to the present embodiment calculates a lower HbA1c value than in a case where the HbA1c value is calculated using equation (3). Consequently, the HPLC apparatus X pertaining to the present embodiment can avoid presenting a higher HbA1c value to the user in a case where it replaces an apparatus that finds the HbA1c value using equation (B). In other words, the HbA1c value calculated by conventional HPLC hemoglobin analyzers for HbA1c value measurement that are predominant in Japan and other East Asian countries and the HbA1c value calculated using equation (1) or equation (2) are about the same, so confusion in users can be prevented.

It will be noted that the measurement unit 102 may find the value of the stable glycohemoglobin (the HbA1c value) by dividing the area of the HbA1c peak that can be fractionated on the chromatogram by a value obtained by subtracting the area of the HbF peak, the area of the HbA2 peak, and the areas of abnormal Hb peaks from the sum total of the areas of the fractionated peaks derived from hemoglobin that can be fractionated on the chromatogram (the total area, i.e., an integral value obtained by integrating the chromatogram). Specifically, the measurement unit 102 may find the value of the stable glycohemoglobin based on equation (3) above. Equation (3) is an example of a second arithmetic equation.

Furthermore, the measurement unit 102 may measure the value of the stable glycohemoglobin using at least either one of the value of the stable glycohemoglobin (the HbA1c value) found using equation (1) or the value of the stable glycohemoglobin (the HbA1c value) found using equation (3). Additionally, the presentation unit 103 may present the value of the stable glycohemoglobin that the measurement unit 102 has found using either equation (1) or equation (3) or the values of the stable glycohemoglobin that the measurement unit 102 has found using equation (1) and equation (3). The presentation unit 103 may also, when presenting the value of the stable glycohemoglobin, present an indication that the value is a conventional type of measurement result in the case of presenting the value found using equation (1) and present an indication that the value is a new type of measurement result in the case of presenting the value found using equation (3). Because values found using equation (3) are higher than values found using equation (1), the presentation unit 103 may also change the threshold for determining that patients are hyperglycemic or suspected of being hyperglycemic between cases where it presents values found using equation (1) and cases where it presents values found using equation (3).

The measurement unit 102 can also calculate the value of HbA2 (the HbA2 value) based on the area of the peak corresponding to the HbA2 component, and the presentation unit 103 may, in accordance with the HbA2 value measured by the measurement unit 102, present a warning that the patient is suspected of having a predetermined condition such as, for example, thalassemia. For example, in a case where the level of HbA2 is 4% or higher, the HbA1c value becomes lower in equation (3) and becomes higher than in equation (1), so the presentation unit 103 may present a warning that the patient is suspected of having thalassemia.

Another method of measuring the HbA1c value will now be described. The measurement unit 102 may find the value of the stable glycohemoglobin (the HbA1c value) represented by A1c% based on equation (1') below. In equation (1'), "A1c" is the area of the HbA1c peak that can be fractionated on the chromatogram, "HbA" is the sum total of the areas of the hemoglobin A peaks including fractions derived from hemoglobin A that can be fractionated on the chromatogram (i.e., not including the areas of the fractionated peaks of HbF, HbA2, and variant hemoglobins), and "$\alpha$" is a coefficient based on the peak area of A2 (the A2 value). It will be noted that coefficient $\alpha$ is greater than 0 and smaller than 1.

$$A1c\% = \frac{A1c}{HbA} \times \alpha \times 100 \quad \cdots \quad (1')$$

Equation (1') above can also be expressed as equation (2') below, and the measurement unit 102 may find the value of the stable glycohemoglobin (the HbA1c value) represented by "A1c%" based on equation (2'). In equation (2'), "A1c" is the area of the HbA1c peak that can be fractionated on the chromatogram, "All Hb" is the sum total of the areas of the fractionated peaks derived from the hemoglobins that can be fractionated on the chromatogram (the total area, i.e., an integral value obtained by integrating the chromatogram), and "$\beta$" is a coefficient based on the areas of the hemoglobin-derived peaks fractionated other than the hemoglobin A peaks. It will be noted that coefficient $\beta$ is greater than 1.

$$A1c\% = \frac{A1c}{\text{All Hb}} \times \beta \times 100 \cdots \quad (2')$$

The correlation between the peak area of A2 and coefficient $\alpha$ in equation (1') and coefficient $\beta$ in equation (2') is determined by a proportional function or a matrix table, and common methods whereby coefficient $\alpha$ in equation (1') and coefficient $\beta$ in equation (2') are found can be exemplified.

Measuring the HbA1c value using equation (1) and equation (3) shows about how much of a difference there is between the two and about how much of a difference there is between healthy subjects and thalassemia patients with high HbA2 values.

Here, it will be assumed that the HbA2 value in healthy subjects is 3.5% and the HbA2 value in thalassemia patients is 7%. In the case of healthy subjects, when the HbA1c value is 5.5% in equation (1) (the equation including the "HbA2" term in the denominator), the HbA1c value becomes 5.7% (+0.2%) when equation (3) (the equation not including the "HbA2" term in the denominator) is used. In the case of thalassemia patients, when the HbA1c value is 5.5% in equation (1), the HbA1c value becomes 5.9% (+0.4%) when equation (3) is used and HbA2 is excluded.

In this way, in equation (1) and equation (3), the HbA1c value that is found becomes higher in equation (1), but in thalassemia patients with high HbA2 values, the difference between the HbA1c value calculated using equation (1) and the HbA1c value calculated using equation (3) becomes larger than in healthy subjects. Consequently, in a case where the measurement unit 102 calculates the HbA2 value and the HbA2 value is equal to or higher than a predetermined threshold, the presentation unit 103 may use the difference between the calculation result using equation (1) and the calculation result using equation (3) to present information such as, for example, a warning that the patient is suspected of having thalassemia.

Next, the action of the control unit 100 will be described.

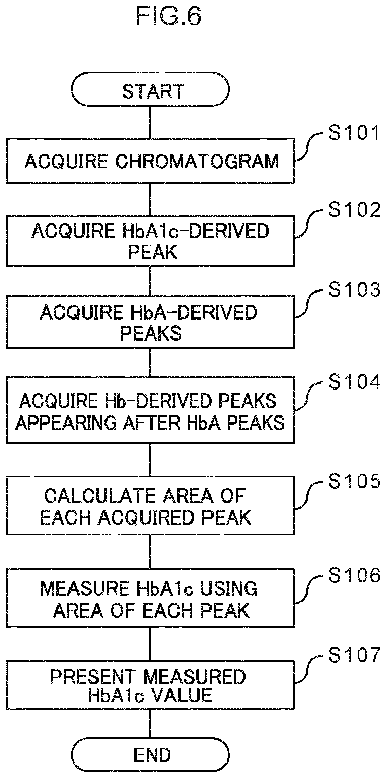

FIG. 6 is a flowchart showing the flow of a stable glycohemoglobin measurement process executed by the control unit 100. The stable glycohemoglobin measurement process is performed by the CPU 100A reading the stable glycohemoglobin measurement program from the ROM 100B or the nonvolatile memory 100D, loading it to RAM 100C, and executing it.

In step S101, the CPU 100A acquires the chromatogram based on the light reception results acquired by the photometer unit 7.

Next, in step S102, the CPU 100A acquires the peak derived from HbA1c from the chromatogram based on the light reception results acquired by the photometer unit 7.

Next, in step S103, the CPU 100A acquires the peaks derived from HbA from the chromatogram based on the light reception results acquired by the photometer unit 7. Here, the peaks derived from HbA include the peak of the HbA1 fraction, the peak of Hb#C fraction, the peak of the HbA1c fraction, and the peak of the HbA0 fraction.

Next, in step S104, the CPU 100A acquires the hemoglobin-derived peaks fractionated after the peaks derived from HbA-for example, the HbA2-derived peak-from the chromatogram based on the light reception results acquired by the photometer unit 7.

Next, in step S105, the CPU 100A calculates the areas of the peaks acquired in steps S102 to S104.

Next, in step S106, the CPU 100A calculates the HbA1c value based on equation (1) using the area of each peak calculated in step S105. The CPU 100A may, when calculating the HbA1c value, perform a calculation using equation (3) in addition to a calculation using equation (1).

Next, in step S107, the CPU 100A presents the HbA1c value calculated in step S106. In a case where the CPU 100A performed a calculation using equation (3) in addition to a calculation using equation (1), it may also present the HbA1c value calculated using equation (3) in addition to the HbA1c value calculated using equation (1).

Because the control unit 100 executes this series of processes, the HPLC apparatus X pertaining to the present embodiment can avoid higher values when measuring stable glycohemoglobin values using a hemoglobin component (e.g., the HbA2 component) that is now able to be fractionated.

In the present embodiment, high-performance liquid chromatography (HPLC) was used to separate hemoglobins and particularly hemoglobins fractionated after the hemoglobin A peaks, such as hemoglobin A2 for example, but embodiments of the present invention may use any separation method as long as the method can separate hemoglobins fractionated after the hemoglobin A peaks, such as hemoglobin A2 for example. Specifically, the present invention may use the method of analyzing hemoglobins in blood disclosed in JP-ANo. 2009-109231, which uses capillary electrophoresis using electrokinetic chromatography based on the principle of cation exchange.

It will be noted that the stable glycohemoglobin measurement process that the CPU executed by reading software (a program) in the above embodiment may also be executed by various types of processors other than a CPU.

Examples of processors in this case include programmable logic devices (PLDs) whose circuit configuration can be changed after manufacture, such as field-programmable gate arrays (FPGAs), and dedicated electrical circuits that are processors having a circuit configuration dedicatedly designed for executing specific processes, such as application-specific integrated circuits (ASICs). Furthermore, the stable glycohemoglobin measurement process may be executed by one of these various types of processors or may be executed by a combination of two or more processors of the same type or different types (e.g., plural FPGAs, and a combination of a CPU and an FPGA, etc.). Furthermore, the hardware structures of these various types of processors are more specifically electrical circuits in which circuit elements such as semiconductor elements are combined.

Furthermore, in the above embodiment, an aspect was described where the stable glycohemoglobin measurement processing program is stored (installed) beforehand in the ROM or the nonvolatile memory, but the program is not limited to this. The program may also be provided in a form in which it is recorded in a recording medium such as a compact disc read-only memory (CD-ROM), a digital versatile disc read-only memory (DVD-ROM), and a universal serial bus (USB) memory. Furthermore, the program may also take a form in which it is downloaded via a network from an external device.

While an embodiment of the present invention has been described in detail above with reference to the accompanying drawings, the technical scope of the present invention is not limited to this example. It is obvious that persons having ordinary skill in the technical field of the present invention can arrive at various changes or modifications within the category of the technical thought set forth in the claims, and it is to be understood that these various changes or modifications also naturally fall within the technical scope of the invention, as defined by the appended claims.

Furthermore, the effects described in the above embodiment are merely illustrative or exemplary, and are not limited to what is described in the above embodiment. In other words, the technology pertaining to the present invention may achieve, together with or instead of the above effects, other effects that will be obvious from the description of the specification to persons having ordinary skill in the technical field of the present invention.

**Claims**

1. A stable glycohemoglobin measurement method whereby a processor (100A) executes a process to:

   separate hemoglobin in blood to acquire a hemoglobin A1c peak derived from stable glycohemoglobin, hemoglobin A peaks including fractions derived from hemoglobin A, and hemoglobin-derived peaks fractionated other than the hemoglobin A peaks; and
   find the value of the stable glycohemoglobin by performing a correction calculation to lower, based on the area of a peak selected from among the hemoglobin-derived peaks fractionated other than the hemoglobin A peaks, a value obtained by dividing the area of the hemoglobin A1c peak by the areas of the hemoglobin A peaks.

2. The stable glycohemoglobin measurement method of claim 1, wherein the processor (100A) executes a process to find the value of the stable glycohemoglobin using a first arithmetic equation where the area of the hemoglobin A1c peak is divided by a value obtained by adding together the areas of the hemoglobin A peaks and the area of the peak selected from among the hemoglobin-derived peaks fractionated other than the hemoglobin A peaks.

3. The stable glycohemoglobin measurement method of claim 2, wherein

   the processor (100A) further acquires a hemoglobin F peak including fractions derived from hemoglobin F and variant hemoglobin peaks corresponding to peaks of variant hemoglobins, and
   the areas of the hemoglobin A peaks are a value obtained by subtracting the area of the hemoglobin F peak and the areas of the variant hemoglobin peaks from the total area of the peaks derived from the hemoglobin.

4. The stable glycohemoglobin measurement method of claim 1, 2 or 3, wherein the selected peak is a hemoglobin A2 peak.

5. The stable glycohemoglobin measurement method of claim 1, wherein the processor (100A) executes a process to:

   further acquire a hemoglobin F peak including fractions derived from hemoglobin F and variant hemoglobin peaks corresponding to peaks of variant hemoglobins; and
   find the value of the stable glycohemoglobin using a second arithmetic equation where the area of the hemoglobin A1c peak is divided by a value obtained by subtracting the area of the hemoglobin F peak, the area of the selected peak, and the areas of the variant hemoglobin peaks from the total area of the peaks derived from the hemoglobin.

6. The stable glycohemoglobin measurement method of claim 5, wherein the processor (100A) executes a process to present at least either of the value of the stable glycohemoglobin found using the first arithmetic equation or the value of the stable glycohemoglobin found using the second arithmetic equation.

7. The stable glycohemoglobin measurement method of claim 5 or 6, wherein the processor (100A) executes a process to:

quantify the value of the hemoglobin corresponding to the selected peak; and,
in a case where the quantified value of the hemoglobin is equal to or higher than a predetermined threshold, present information based on the difference between the value of the stable glycohemoglobin found using the first arithmetic equation and the value of the stable glycohemoglobin found using the second arithmetic equation.

8. The stable glycohemoglobin measurement method of any preceding claim, wherein the processor (100A) executes a process to separate the hemoglobin in the blood using liquid chromatography that is capable of separating the hemoglobin A1c peak, the hemoglobin A peaks, and the hemoglobin-derived peaks.

9. A stable glycohemoglobin measurement apparatus comprising:

an acquisition unit (101) configured to separate hemoglobin in blood to acquire a hemoglobin A1c peak derived from stable glycohemoglobin, hemoglobin A peaks including fractions derived from hemoglobin A, and hemoglobin-derived peaks fractionated other than the hemoglobin A peaks; and
a measurement unit (102) configured to find the value of the stable glycohemoglobin by performing a correction calculation to lower, based on the area of a peak selected from among the hemoglobin-derived peaks fractionated other than the hemoglobin A peaks, a value obtained by dividing the area of the hemoglobin A1c peak by the areas of the hemoglobin A peaks.

10. A non-transitory recording medium storing a program that is executable by a computer to perform a process, the process comprising:

separating hemoglobin in blood to acquire a hemoglobin A1c peak derived from stable glycohemoglobin, hemoglobin A peaks including fractions derived from hemoglobin A, and hemoglobin-derived peaks fractionated other than the hemoglobin A peaks; and
finding the value of the stable glycohemoglobin by performing a correction calculation to lower, based on the area of a peak selected from among the hemoglobin-derived peaks fractionated other than the hemoglobin A peaks, a value obtained by dividing the area of the hemoglobin A1c peak by the areas of the hemoglobin A peaks.

FIG.1

EP 4 343 323 A1

# FIG.2

# FIG.3

FIG.4

# FIG.5

A0/A2/E/D

S,C

A1c

F    #C

A1

LIQUID A     LIQUID C    LIQUID B

# FIG.6

```
         ┌─────────────┐
         │    START    │
         └─────────────┘
                │
    ┌───────────────────────────┐   ┌ S101
    │   ACQUIRE CHROMATOGRAM     │
    └───────────────────────────┘
                │
    ┌───────────────────────────┐   ┌ S102
    │   ACQUIRE HbA1c-DERIVED    │
    │           PEAK            │
    └───────────────────────────┘
                │
    ┌───────────────────────────┐   ┌ S103
    │    ACQUIRE HbA-DERIVED     │
    │           PEAKS           │
    └───────────────────────────┘
                │
    ┌───────────────────────────┐   ┌ S104
    │  ACQUIRE Hb-DERIVED PEAKS  │
    │  APPEARING AFTER HbA PEAKS │
    └───────────────────────────┘
                │
    ┌───────────────────────────┐   ┌ S105
    │      CALCULATE AREA OF     │
    │    EACH ACQUIRED PEAK      │
    └───────────────────────────┘
                │
    ┌───────────────────────────┐   ┌ S106
    │     MEASURE HbA1c USING    │
    │     AREA OF EACH PEAK      │
    └───────────────────────────┘
                │
    ┌───────────────────────────┐   ┌ S107
    │     PRESENT MEASURED       │
    │        HbA1c VALUE         │
    └───────────────────────────┘
                │
         ┌─────────────┐
         │     END     │
         └─────────────┘
```

Europäisches
Patentamt
European
Patent Office
Office européen
des brevets

# EUROPEAN SEARCH REPORT

Application Number

EP 23 17 4366

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Lynn Bry ET AL: "Effects of Hemoglobin Variants and Chemically Modified Derivatives on Assays for Glycohemoglobin Hb Variants and Derivatives", <br> , <br> 1 February 2001 (2001-02-01), XP055644960, Retrieved from the Internet: URL:http://clinchem.aaccjnls.org/content/clinchem/47/2/153.full.pdf [retrieved on 2019-11-21] * the whole document * <br> ----- | 1-10 | INV. <br> G01N30/88 <br> G16H50/20 <br> G01N30/86 |
| X | Anonymous: "HbA1c and interference due to hemoglobin disorders", <br> , <br> 1 January 2019 (2019-01-01), XP093128917, Retrieved from the Internet: URL:www.diagnostics.roche.com /resource-center/ cps [retrieved on 2024-02-07] | 1-6,8-10 | |
| Y | * p. 12-14; p. 4-6 * <br> ----- | 7 | TECHNICAL FIELDS SEARCHED (IPC) <br> G01N <br> G16H |
| Y | CHANDRASHEKAR VANI: "Hb A1c Separation by High Performance Liquid Chromatography in Hemoglobinopathies", SCIENTIFICA, vol. 2016, 1 January 2016 (2016-01-01), pages 1-4, XP093128922, US ISSN: 2090-908X, DOI: 10.1155/2016/2698362 Retrieved from the Internet: URL:https://downloads.hindawi.com/journals /scientifica/2016/2698362.pdf> * p. 2; Fig. 2, 3 * <br> ----- | 7 | |

The present search report has been drawn up for all claims

| Place of search | Date of completion of the search | Examiner |
|---|---|---|
| Munich | 13 February 2024 | Schalich, Juliane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................
& : member of the same patent family, corresponding document

EPO FORM 1503 03.82 (P04C01)

**REFERENCES CITED IN THE DESCRIPTION**

**Patent documents cited in the description**

- JP 2017198666 A
- JP 2001228133 A
- JP 2009109231 A
- JP 2014235023 A
- JP 2021056174 A